# EUROPEAN PATENT APPLICATION

(11) **EP 4 521 222 A1**
(43) Date of publication of application: **12.03.2025**
(21) Application number: 23195747.3
(22) Date of filing: 06.09.2023
(51) Int. Cl.: G06F 3/04842, G06F 3/04845, G06F 3/01, G16H 50/20, G16H 30/40

(54) **MEDICAL IMAGE PROCESSING SYSTEM AND METHOD**

(71) Applicant: Koninklijke Philips N.V., 5656 AG Eindhoven (NL)
(72) Inventor: PAPACONSTADOPOULOS, Pavlos, 5656 AG Eindhoven (NL); LAVES, Max-Heinrich Viktor, 5656 AG Eindhoven (NL); PAWAR, Anil, 5656 AG Eindhoven (NL); ADAK, Saptakatha, 5656 AG Eindhoven (NL); MYSORE SIDDU, Dinesh, 5656 AG Eindhoven (NL)
(74) Representative: Philips Intellectual Property & Standards

(57) **Abstract**

A medical image processing system and method involves receiving an image of a medical scan together with location information indicating a location of the image at which a user's attention is directed during a period of analysis of the image by the user. Time periods are derived during which the user's attention is directed to each image location and a time period representation is output which represents the time periods and their associated image locations. This may be used to assist in the manual inspection of medical images, or it may be used to assist in the training of machine learning based anomaly detection.

## Description

### FIELD OF THE INVENTION

This invention relates to medical imaging systems, in particular for processing images received from a medical scanner.

### BACKGROUND OF THE INVENTION

Medical imaging is a crucial component of the clinical diagnostic and treatment workflow. In several clinical settings, a medical image scan, such as a CT, MRI or PET scan, is a requirement for the clinician to identify and diagnose the clinical problem. Often, multiple doctors will revisit these image scans depending on the workflow step. For example, during the diagnostic phase, a physician will order a CT or MR scan for assessing the probability of a disease or pathological condition under investigation. Lung cancer will be used below as an example of a disease under investigation. However, even in respect of lung examination, there may be other lung disease signatures such as Atelectasis, Cardiomegaly, Effusion, Infiltration, Mass, Nodule, Pneumonia, Pneumothorax, Consolidation, Fibrosis, Pleural Thickening. Continuing with the example of a lung cancer investigation, if the doctor identifies and diagnoses lung cancer, the case will go to an oncologist who will review the diagnostic scan to decide a treatment path. If radiotherapy is decided a radiation oncologist will review and possibly request a new CT or MR scan for treatment planning purposes.

Medical image scans are usually performed to fully cover a specific anatomical region. Taking the example of of lung cancer, a protocol will be followed, which dictates, for example, that the patient will be scanned from the lower abdomen to upper shoulder-mandible area. These scan limits are needed mostly for practical reasons. For example, reproducibility of patient set up is crucial if treatment will be followed later.

The physician however will naturally draw their attention to specific regions of the medical image, which are the most suspicious for cancer. After identifying the region, the clinician will report where the cancer was found in the lungs (e.g., diagnostic radiology) or segment the gross tumor volume (GTV), the visible extension of the tumor (e.g., radiation oncology).

The clinically relevant region is more specific spatially than the original scan dimensions. For example, the lower abdomen or mandible regions are no longer relevant after a cancer has been identified in the lung region, for example, close to the heart (approximately in the middle of the scan). To some extent, the initial scan borders already indicated a very general area of interest and excluded other areas. In the lung case, most of the head region is not included in the scan as it is considered not to contribute more information regarding the possibility of cancer in the lungs.

The exclusion of regions which are present in the initial scan borders but are not of clinical interest comes from clinical experience, and it is important not to miss important regions which may still contain useful information for diagnostic or treatment purposes.

In recent years, artificial intelligence (AI) / deep learning (DL) models have created a revolution in medical imaging and medical image segmentation activities, continuously creating new breakthroughs in segmentation accuracy. AI will be used to cover all machine learning models, such as hand-crafted features-based models or deep learning models. To train such models, many medical images (e.g., thousands) generated by scanners such as CT or MRI, and significant computational resources (e.g., GPUs, memory) are required. Multiple image modalities are also now commonly used to increase the anatomical and functional information learned by the AI model during training. It is well known that the performance of such AI models is improved if more relevant cases are provided including the specific regions of importance. Indeed, researchers often try to aid the model training by cropping around areas of interest and let the model focus on features in those local regions. Careful selection of the areas of clinical regions also aids the computational resources required for DL model training, which usually means very high memory requirements. If, however, the image does not need to be that large it would reduce such requirements.

There is thus a problem that during the clinical workflow, medical image scans are used for clinical decisions and/or for AI/DL model training without fully utilizing prior knowledge of the clinically relevant regions. Improvement is needed in the way medical images are processed along the diagnostic and treatment workflow.

### SUMMARY OF THE INVENTION

According to examples in accordance with an aspect of the invention, there is provided a medical image processing system, comprising a processor which is configured to:
receive at least one image of a medical scan comprising an array of image locations;
receive location information indicating an attention location of the image at which a user's attention is directed at each point in time during the user's analysis of the image;
derive time periods during which the user's attention is directed to each image location of the array of image locations; and
output a time period representation comprising time periods and their associated image locations.

This processing system performs a time period analysis for medical scan images and outputs a time period representation. The time period analysis identifies how long a user (clinician) spends studying different parts of the image, together covering the full image. This studying of a part of an image for example involves looking at that part of the image, and annotating or modifying the image (e.g. to draw lines for a dimension measurement such as a diameter, to zoom in to obtain more insights on the suspicious region, or to hover a mouse pointer over a suspicious region to change the window levelling (hence brightness) and spend more time to analyze the region) using a user input device. This gives an indication of the parts of the image that are considered relevant by the clinician to the diagnosis that is being made.

A time period representation is generated. This enables a duration of study of each image location, at least relative to the other image locations, to be indicated.

The time period representation may then be used to assist clinicians, for example clinicians who need to study an image that has already been studied by a clinician in a different field. The time period representation (or underlying data) may also be used in the training of machine learning models in their decision making by highlighting regions of high clinical relevance to the task at hand. The array of image locations for example comprises array of image pixels, and timing information is derived for each image pixel. There is thus a one-to-one spatial mapping between the time period analysis and the scan image, with the time period analysis providing a per-pixel (relative) timing value. However, the image locations may be larger than the pixels of the medical scan image, so that the resolution of the time period analysis can be lower than the resolution of can image. In such a case, each image location may be considered to be a smallest area of interest.

The processor is for example configured to receive the location information from a visual navigation accessory, such as a mouse or stylus.

While analyzing the image, the user will make annotations or selections using the mouse or stylus at the location of current interest to the user. Thus, the navigation information indicates the areas of interest to the user.

The processor is for example configured to receive the location information from a gaze tracking system. This provides an alternative way to monitor the part of an image that is of interest to the user.

The time period representation for example comprises a time map image. It may take the form of a heat map, wherein different heats (e.g., colors of an image) represent different analysis durations for the different image locations. The time map image comprises a visual representation of the relative time periods, for example using color or grey scale coding, to provide an intuitive guide to the relative importance of different parts of the image, as perceived by a user who has previously analyzed the image. The time map image may be a non-numeric indication of the time values so that a more intuitive representation is provided. However, numeric values may be presented instead or as well.

The system for example further comprises a display for outputting a combination image which combines the medical scan image and the time map image. The time map image is for example presented as overlay to the medical scan image.

The system may further comprise the visual navigation accessory mentioned above.

The system may further comprise an anomaly detection system for detecting a target anomaly in a medical scan image for identifying abnormal tissue using the medical scan image and the derived time periods. An anomaly detection algorithm is for example a machine learning algorithm, which is trained using both the medical scan image and the time period representation (or underlying time period information), and which processes the medical scan image and the time period representation to provide information to the user.

In addition to providing the anomaly detection result, it may provide other information and warnings. For example,. During the inference, clinicians can use the anomaly detector to detect abnormal areas that exist in an image and prompt a clinician's attention to these areas.

The anomaly detection system is for example configured to:
determine time period information for the current attention location, while the user is delineating an image; and
provide a warning if the determined time period information suggests the attention location is away from the anomaly.

For example, as a physician is delineating an area of interest, if they start moving into less visited areas (hence with short time period) the anomaly detector can locate the abnormal tissue and raise a warning flag.

The invention also provides a method of processing a medical scan image comprising an array of image locations, comprising:
receiving location information indicating an attention location of the medical scan image at which a user's attention is directed at each point in time during the user's analysis of the image;
deriving time periods during which the user's attention is directed to each image location of the array of image locations; and
outputting a time period representation comprising the time periods and their associated image locations.

The method for example comprises receiving the location information from a visual navigation accessory such as a computer mouse or stylus or remote pointer device, or indeed any input device enabling a user to provide an input in respect of a visually chosen part of an image. The method may also comprise displaying a combination image which combines the medical scan image and the time period representation, wherein the time period representation comprises a time map image.

The invention also provides a computer program comprising computer program code means which is adapted, when said program is run on a computer, to implement the methods defined above.

The invention also provides a method of training a machine learning model for detecting anomalies in medical scan images, comprising:
training the model using medical scan images and time period analyses,
wherein the medical scan images each comprise an array of image locations and wherein the time period analyses comprise time periods during which a user's attention was directed to each image location of the array of image locations of an image during a user's analysis of the image.

This method makes use of the time period analyses generated by the system defined above to assist in the training of a machine learning model. The training may use the time period representations, or the data underlying those representations, i.e., the derived time periods.

In particular, the time period analyses provide additional context about the medical scan images, and this may be used to save processing effort for the machine learning algorithm in detecting target anomalies.

The training for example comprises applying weights to different image locations based on the time periods.

The trained model may also be used to provide time period information for a new medical scan image. For example, time periods recorded on each medical image may be translated to a probability of visiting a location (e.g., 100% indicates that this location is always looked at and 0% if it is never looked at). Given this information, outliers in future images can be detected, for example to detect that a user is looking at an area where it is improbable to find anything of interest.

Thus, time period analyses are not only of interest when one clinician views a particular image which has already been viewed by another clinician, but also may be used to train the algorithm to learn and predict which locations will be of most interest in a new medical scan image (i.e., for the first analysis by a user).

The invention also provides a machine leaning model which has been trained using the method as defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a medical image processing system;
Fig. 2 shows a method of processing a medical scan image; and
Fig. 3 shows a method of training a machine learning model for detecting anomalies in medical scan images.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a medical image processing system and method that involves receiving an image of a medical scan together with location information indicating a location of the image at which a user's attention is directed during a period of analysis of the image by the user. Time periods are derived during which the user's attention is directed to each image location and a time period representation is output which represents the time periods and their associated image locations. This may be used to assist in the manual inspection of medical images, or it may be used to assist in the training of machine learning based anomaly detection.

This invention aims to aid clinicians and/or AI machine learning models in their decision making, by highlighting regions of high clinical relevance for the task. For the clinician, this addresses the problem of identifying where to look for an anomaly (e.g., tumor) by utilizing the information from past decisions and/or prior steps in the workflow.

In the known procedure, when a clinician analyzes a scan image, every time a new scan is performed, the full image scan is taking into consideration from the beginning, even if some other physician may have already identified the region of interest in a previous step. For example, in oncology the diagnostic radiologist may identify cancer somewhere and moves the case to oncology, but the radiation oncologist afterwards looks at the full image again (and often requests a completely new image) and delineates the tumor from the beginning. It is known in clinical practice that often radiation oncologists will delineate tumors in different regions from where the radiologist found them a step before. This may have detrimental effects to the treatment outcome as it may lead to errors and mistreatments.

For the AI models, the invention addresses the problem of assisting the identification of the locations of anomalies or improving the training of the model. Known AI models currently utilize the full image scan for training, even if some regions are not clinically relevant at all. Even though the model will likely eventually find the clinically relevant regions, this may come with a performance and resources cost. In addition, if the data are not well-distributed, the model may use features for its predictions that have no clinical meaning, but they do preserve some correlations with the target tasks. Such regions are difficult to be identified without some prior clinical knowledge input into the model training.

The invention is based on tracking a physician's behavior during image inspection and in some embodiments image segmentation, thus creating a time period analysis which identifies regions of high clinical relevance and regions of low clinical relevance. Such an analysis can be used immediately in clinical practice when an image is passed from one doctor to the next or it may be used as an extra input channel to AI models to weigh more areas of high clinical relevance and less areas of low clinical relevance. As mentioned above, an example of passing images would be radiologist passing images to a radiation oncologist. The information indicating where the radiologist visited the image is useful to avoid errors and ensure consistency in practice. Another example is images being passed from a pathologist to a medical oncologist.

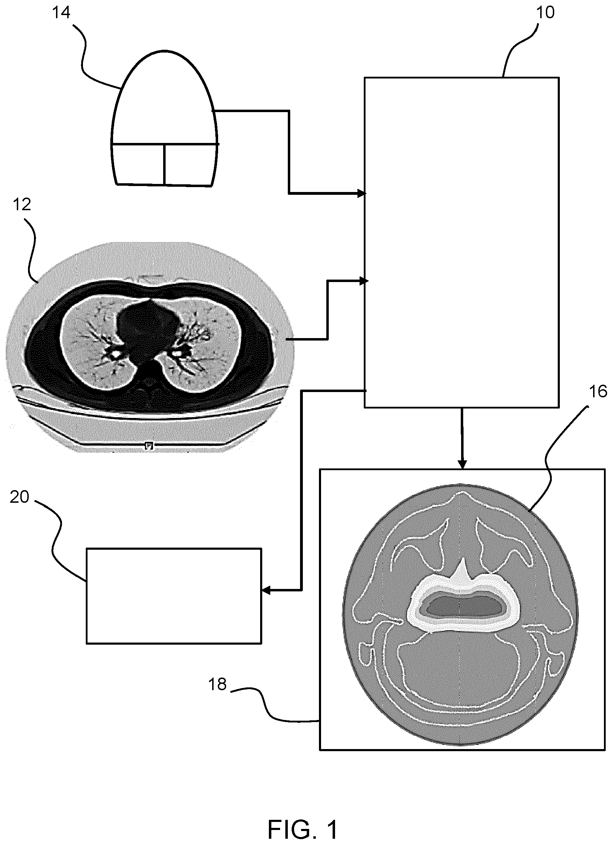

Fig. 1 shows a medical image processing system comprising a processor 10 which receives an image 12 of a medical scan comprising an array of pixels (i.e., image locations). Location information is also received during a period of analysis of the image 12. The location information indicates a location of the image at which a user's attention is directed at each point in time during the period of analysis. In the example of Fig. 1, the location information is received from a computer mouse 14. More generally, the location information may be received from any visual navigation accessory, such as a stylus. The processor has a module for capturing mouse (or other accessory) movements on the screen and for translating relative positions to the image coordinate system. The period of analysis is determined by the processor, by triggering a start session and an end session for the use of the navigation accessory.

In another example, the location information is received from a gaze tracking system. Multiple modalities may be combined (e.g., mouse tracking and gaze tracking) for determining a location of the image which is being assessed by the user.

While analyzing the scan image, the clinician makes annotations or selections using the mouse or stylus and hence the location information indicates the areas of interest to the clinician. The clinician for example may draw over a part of the image, for example to draw lines for a dimension measurement such as a diameter. The clinician may use a pointer to zoom in on a particular area, or to hover a mouse pointer over an area to change the display characteristics such as window levelling. All of these actions involve a mouse or other pointer being located at a particular region of the image which is of interest to the user.

The processor 10 has a timer so that it can derive time periods during which the user's attention is directed to each pixel (i.e., each image location).

The time periods for the multiple pixels together define a time period analysis. This analysis provides the time periods (typically in relative terms rather than absolute terms) for each associated pixel.

In a preferred example, the time period analysis is a graphical visual representation of the relative time periods, for example using color or grey scale coding. Fig. 1 shows a graphical representation which will be termed a time map image 16. It is presented as an output on a display 18. In one example, the time map image 16 is provided as an overlay to the original scan image 12.

The time period analysis identifies how long a user (clinician) spends studying different parts of the image, and it thereby informs the parts of the image that are considered relevant by the clinician to the diagnosis that is being made.

Fig. 1 also shows an anomaly detection system 20 for detecting a target anomaly in a medical scan image. The anomaly detection system uses an algorithm that has been trained using the time period analysis, as explained further below. The anomaly detection system may use as inputs only the scan image, but the training of the algorithm using the time period analysis saves processing effort. Alternatively, the anomaly detection system may take as inputs both the scan image as well as a time period analysis associated with that scan image, when there has already been a clinician's analysis of that image.

For example, by monitoring the location of interest of a user, if the user starts moving to a location which was previously not of interest, the anomaly detection system can provide a warning that this location may not be of interest. For example, a user can then understand that they may be considering a particular location to show a tumor and/or disease signature and/or a pathological feature, but this is an area where such features do not usually appear.

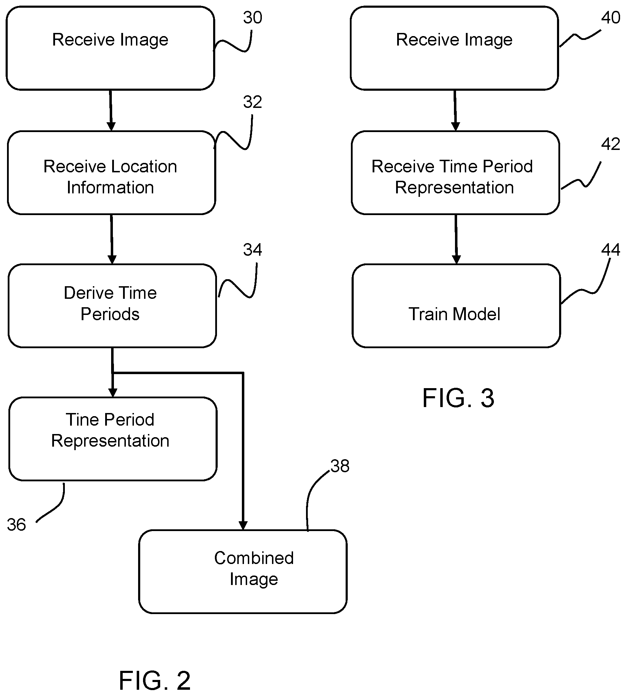

Fig 2 shows a method of processing a medical scan image comprising an array of pixels.

A medical scan is received in step 30 from a medical scanner such as a CT scanner, PET scanner or MRI scanner. In step 32, location information is received indicating a location of the image at which a user's attention is directed at each point in time during a period of analysis of the image by the user.

Time periods during which the user's attention is directed to each pixel are obtained in step 34. This information is saved in memory and a time period analysis is generated and output in step 36. The time period analysis may instead be output as part of a combined image as shown in step 38.

The use of the system and method of Fig. 2 will be explained with reference to lung cancer diagnosis. After a diagnostic radiologist has ordered a CT scan, the radiologist will use the mouse pointer (or other computer accessory commonly used for inspecting and delineating regions of interest on a medical image e.g., a stylus) to navigate in the regions of interest within the image scan. As the mouse pointer moves across various pixels on each image plane, the processor records the time the pointer remains on each pixel. For example, in the case of the lung patient, the physician will quickly move forward through the initial slides of the mandible and upper shoulders, which have little clinical relevance and focus their attention to the slides with the lung organs, with more clinical relevance. As the physician moves with the mouse more time will be recorded in the areas that a tumor is suspected.

The clinicians will for example be looking at a 3D volume, in the form of a series of 2D slice images. Location information and time period information is collected for every image in the series for a patient study. Each patient study typically contains multiple series (i.e., multiple 3D volumes), and each 3D volume contains a set of 2D image slices. For each of those 2D slices, the location and time period information are collected.

A physician could in theory delineate the tumor, but this is not common practice for radiologists, who mostly focus on identifying the tumor and reporting it in a medical report.

When the session is over, and the radiologists closes the image viewer, the time spent per pixel is saved together with the image. This will be used to generate a time period analysis, which for example comprises a new image in the form of a map of the time spent per time per pixel. This will be termed a time map image. Since the time map image is generated from the image itself, there is 1:1 association of the two images. The time map image may also be saved as a new image following any suitable international standard in medical imaging (e.g. DICOM). In addition to the time per pixel information, more information can be included in the data file of the time map image, such as the start session time, end session time, physician name etc.

Assuming the lung patient moves into the treatment phase, a radiation oncologist will inspect the diagnostic CT scan or order a new CT scan for treatment planning purposes. Together with the original images, the radiation oncologist can also consult the time map image. Using a common image viewer software, the radiation oncologist will be able to inspect the original image together with the time map image. In some embodiments, the original image and time map image are overlaid, in some other embodiments they are represented side-by-side. Other representations are envisioned within the context of the present invention.

The time map image can be presented overlaid on the original image in the form of a colored heat map. "Hot" areas (e.g., red) may indicate pixels that the previous physician visited, while "cold" areas will indicate areas that were not visited much. In this way, the radiation oncologist will appreciate the areas of clinical relevance from the previous physician and utilize this information to segment the tumor more accurately. A mismatch between radiology and radiation oncology will be reduced and the possibility for gross errors is thereby reduced.

The time map image may be presented in different ways. Any representation which gives a viewer an intuitive indication of the relative time durations may be used. This may involve the use of colors (as explained above), contours, words, numbers or symbols. The time map image may be combined with the original image in such a way that the information of interest in the original image is not obscured. Alternatively, the time map image may be alongside the original image.

Fig. 3 shows a method of training a machine learning model for detecting anomalies in medical scan images. The machine learning model is a deep learning model for medical image segmentation, with multiple input channels. A scan image is received in step 40, and a time period analysis is received in step 42. The model is trained in step 44 using the medical scan images and time period analyses. The training uses both normal anatomy images and the images where a clinician spends time reviewing. During the training, the machine learning model learns the areas where the clinician has spent more time during the interpretation and the corresponding abnormality that exists in the given anatomical images.

The time period analyses provide additional context about the medical scan images as explained above and this may be used to save processing effort for the machine learning algorithm in detecting target anomalies.

The training for example comprises applying weights to different image locations based on the time periods. More weight can be applied to the areas where physicians spent more time. A new input channel is used during the model training, for receiving the time map image. The time map image can serve as weights per pixel for the model, forcing the model to look for features mostly in these areas. Alternatively, the time map image can be used as a predictive target during model training. In particular, given enough examples, the model will be able to predict the most visited areas and the less visited areas on a new image scan without any time map or segmentation provided.

As shown above, the time map image can also be used by an anomaly detector algorithm. While the physicians are delineating the images, if they start moving into less visited areas, the anomaly detector can locate the abnormal tissue and raise a warning flag. In such a way the physician can appreciate if what they observe as tumor is in fact what is commonly observed in those areas. Even if not an error, such flags could be useful to increase the awareness that this is an area where usually tumors do not appear.

Multiple physicians or multiple clinics could share their time map images in a common repository or cloud server or via any other means of sharing information. The clinics may compare their average most visited and less visited areas on the image scan per tumor site (e.g., lung). Such information can be useful to create insights on tumor growth among the population. In the case that some areas are never visited, the training system could be instructed not to utilize those areas at all during AI model training. In such a way, computational resources needed for training can immediately benefit by the reduced image sizes.

Thus, there may a data store which stores medical scan images together with time periods during which a user's attention was directed to each image location during a user's analysis of the image. This data store stores image data and time information for multiple physicians. By grouping the data into different types of anomaly under investigation (and hence different scan areas), information relating to the population as a whole may be derived.

An embodiment thus also provides a data structure comprising a medical scan image comprising image data for an array of image locations and time period information comprising time periods during which a user's attention is directed to each image location of the array of image locations during the user's analysis of the image.

The data store mentioned above then comprises multiple data structure samples for multiple images and for multiple physicians (i.e., multiple users).

The machine learning algorithm may be any suitable algorithm that processes input data in order to produce or predict output data. Here, the input data comprises the scan images and the time period analyses, and the output data comprises a diagnostic conclusion as to the presence or absence of a target anomaly, e.g., a particular tumor.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g., the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "sensor" or "system." Furthermore, aspects of the present invention may take the form of a computer program embodied in one or more computer readable medium(s) having computer executable code embodied thereon. Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. A computer readable signal medium may include a propagated data signal with computer executable code embodied therein, for example, in baseband or as part of a carrier wave.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A medical image processing system, comprising a processor (10) which is configured to:
(30) receive at least one image of a medical scan comprising an array of image locations;
(32) receive location information indicating an attention location of the image at which a user's attention is directed at each point in time during the user's analysis of the image;
(34) derive time periods during which the user's attention is directed to each image location of the array of image locations; and
(36, 38) output a time period representation comprising time periods and their associated image locations.

2. The system of claim 1, wherein the processor (10) is configured to receive the location information from a visual navigation accessory device, such as a mouse (14), stylus or gaze tracking system.

3. The system of any one of claims 1 to 2, wherein the time period representation comprises a time map image (16) which provides a representation of relative time values between different image locations, such as a heat map.

4. The system of claim 3, further comprising a display (18) for outputting the time map image (16) and/or a combination image which combines the medical scan image (12) and the time map image (16).

5. The system of claim 4, wherein the processor is configured to control the display to generate a combination image comprising a heat map overlaid over the medical scan image.

6. The system of any one of claims 4 to 5, wherein the processor is further configured to perform image segmentation of the medical scan image and to control the display to output the segmented medical scan image.

7. The system of any one of claims 1 to 6, further comprising an anomaly detection system (20) for detecting a target anomaly in a medical scan image using the medical scan image and the derived time periods.

8. The system of claim 7, wherein the anomaly detection system is configured to:
determine time period information for the current attention location, while the user is delineating an image; and
provide a warning if the determined time period information suggests the attention location is away from the anomaly.

9. A method of processing a medical scan image comprising an array of image locations, comprising:
(32) receiving location information indicating an attention location of the medical scan image at which a user's attention is directed at each point in time during the user's analysis of the image;
(34) deriving time periods during which the user's attention is directed to each image location of the array of image locations; and
(36, 38) outputting a time period representation comprising the time periods and their associated image locations.

10. The method of claim 9, comprising receiving the location information from a visual navigation accessory such as a computer mouse or stylus.

11. The method of any one of claims 9 to 10, comprising (38) displaying a combination image which combines the medical scan image and the time period representation, wherein the time period representation comprises a time map image (16).

12. A computer program comprising computer program code which is adapted, when said program is run on a computer, to implement the method of any one of claims 9 to 11.

13. A method of training a machine learning model for detecting anomalies in medical scan images, comprising:
(44) training the model using medical scan images and time period analyses,
wherein the medical scan images each comprise an array of image locations and wherein the time period analyses comprise time periods during which a user's attention was directed to each image location of the array of image locations of an image during the user's analysis of the image.

14. The method of claim 12, wherein the training comprises applying weights to different image locations based on the time periods.

15. A machine leaning model which has been trained using the method of any one of claims 13 to 14.
